# EUROPEAN PATENT SPECIFICATION

(11) **EP 0 826 026 B1**
(45) Date of publication and mention of the grant of the patent: **09.05.2001**
(21) Application number: 95940272.8
(22) Date of filing: 13.12.1995
(51) Int. Cl.: C11D 3/50, C11D 3/12, C11D 17/00, A61K 7/46, A61K 7/48

(54) **PARTICLES CONTAINING PERFUME**
PARFÜMHALTIGE TEILCHEN
PARTICULES CONTENANT DU PARFUM

(30) Priority: 11.01.1995 GB 9500536
(43) Date of publication of application: 04.03.1998
(73) Proprietor: UNILEVER PLC, London EC4P 4BQ (GB); UNILEVER N.V., 3013 AL Rotterdam (NL)
(72) Inventor: ARAYA, Abraham, Wirral Merseyside L63 3HY (GB); CHAPPLE, Andrew, Paul, Gwynfryn Near Wrexham Clwyd LL11 5UN (GB); MADDISON, Christopher, South Wirral Cheshire L66 1RE (GB)
(74) Representative: Fransella, Mary Evelyn
(86) International application number: EP9505000
(87) International publication number: WO9621719

(56) References cited:
- EP-A- 0 149 264
- EP-A- 0 521 635
- EP-A- 0 535 942
- EP-A- 0 684 301

## Description

This invention relates to perfume-containing particles suitable for use in fabric laundering detergent compositions.

### Background of the Invention and Summary of the Prior Art

There have been a number of proposals for absorbing perfume onto a solid carrier material, and various reasons for doing so. In a number of instances, the objective of such proposals has been merely to convert liquid perfume into a solid form which can more readily be incorporated into another product. It has been asserted that such carrier substances may serve to protect the perfume from loss during storage or protect it from contact with other constituents of a composition.

Examples of disclosures of such carriers for perfume are GB-A-1306924, US-A-3449266, US-A-3909461, US-A-4536315, US-A-4539135, US-A-4713193, GB-A-2066839, EP-A-332259, EP-A-332260 and JP-A-63/79662.

EP-A-535942 and EP-A-536942 are concerned with carrier materials which serve to reduce the vapour pressure of absorbed perfume.

As pointed out in those documents, a carrier material which reduces the perfume vapour pressure over the solid carrier but releases the perfume on contact with water can be useful when perfuming a concentrated laundry detergent composition or fabric softening composition.

These concentrated products are used in relatively small quantity by the consumer and therefore need to contain a higher concentration of perfume than is otherwise customary, in order to provide a normal quantity of perfume when added to a wash or rinse liquor for use. However, the presence of such an enhanced concentration of perfume in such products can give them an unpleasantly strong odour. As disclosed in the above mentioned European published application, the use of a carrier material which reduces the vapour pressure of the perfume can serve to reduce the perfume odour of the product to a satisfactory level, whilst still providing the desired quantity of perfume in a wash liquor or rinse liquor at the time of use.

Another possible utilisation of carriers which reduce the vapour pressure of perfume is to permit a change in fragrance when the laundry detergent or fabric softening composition is added to water to form a wash or rinse liquor. To achieve this, some perfume ingredients would be absorbed onto the carrier while others were not. Until the composition is added to water at the time of use, the perfume constituents on the carrier would have their vapour pressure and consequent perceptibility reduced relative to the other ingredients which were not so absorbed.

In these prior disclosures, the carrier substance has generally been a material which was inert. It was desirable to achieve a high loading of perfume onto the carrier solid so as to keep to a minimum the quantity of carrier solid which was required.

Our EP-A-535942, teaches that the carrier material should have a substantial volume of pores, having a pore diameter in a range from 7 to 50 angstroms.

Materials which can fulfil this criterion are some silicas and zeolite Y. Zeolite Y does not have sufficient ion exchange capacity for use as a detergency builder. By contrast, zeolite 4A which does sufficient have ion exchange capacity and is useful as a detergency builder, does not fulfil this criterion for a volume of pores in the size range 70 to 500 nanometres (7 to 50 angstroms). A comparative example in EP-A-535942 shows that zeolite 4A can serve as a carrier for perfume, but gives only a small reduction in the vapour pressure of the perfume even in the absence of other ingredients of a detergent composition.

Our EP-A-384070 published on 29 August 1990 discloses another type of zeolite which is able to function as a detergency builder. It is a form of zeolite P, more specifically it is defined in EP-A-384070 as alkali metal aluminosilicate of the zeolite P type having a silicon to aluminium ratio not greater than 1.33, preferably not greater than 1.15. This material can be called "maximum aluminium zeolite P", or "zeolite MAP". A preferred process for making it is the subject of EP-A-565364. EP-A-521635 discloses zeolite MAP as a carrier for liquid material, especially nonionic surfactant and mentions that it can also be used to carry other materials including perfume. In this document a preference is stated for a quantity of carried liquid which is at least 10% of the quantity of carrier, but more preferably at least 35%.

In EP-A-684 301, which is a prior document under Article 54(3) only, Example A discloses a composition, including spray-dried particles containing zeolite MAP onto which perfume is sprayed, which contains overall:
zeolite MAP :18%
co-polymer of 1:4 maleic/acrylic acids: 4%
diethylene triamine penta (methylene phosphonic acid): 0.5%
brightener: 0.2%
magnesium sulphate: 0.4%
perfume: 0.1%
C₁₄ - C₁₅ alcohol ethoxylate with average 7 ethoxy groups per mole of alcohol: 4%
silicone suds suppressor: 0.5%
tallow alcohol having average 11 ethylene oxide groups per mole of alcohol: 1%
percarbonate bleach: 18%
tetraacetyl ethylene diamine: 5%
trisodium citrate dihydrate: 10%
linear C₈₋₁₁ alkylbenzene sulphonate: 7%
sodium C_{16 - 18} alkyl sulphate: 2%
C_{12 - 15} alcohol ethoxysulphate having an average of 3 ethoxy groups per mole: 0.2%
sodium carboxymethyl cellulose: 0.3%

- sodium carbonate:: 15%
- sodium bicarbonate:: 2%
- additional perfume as 40% perfume in dextrin capsules:: 0.2%
- Savinase:: 1.5%
- and other ingredients to a balance of:: 100%.

This composition is a particulate composition of density 700 gm/litre.

### Summary of the Invention

We have found that this zeolite MAP can serve as a useful carrier for perfume. Although it does not meet the pore size/volume requirements of our EP-A-535942 mentioned above, we have surprisingly found that if the concentration of perfume on this zeolite is kept within a modest range, a reduction in perfume vapour pressure is obtained.

Because the zeolite can itself be incorporated in a detergent composition in substantial amounts in order to function as a detergency builder, and thus perform a useful function in the wash liquor, it is useful as a perfume carrier even though the concentration of perfume on the zeolite is not high.

Broadly, therefore, this invention, provides alkali metal aluminosilicate of the zeolite P type having a silicon to aluminium ratio not greater than 1.33, and having perfume absorbed thereon in a quantity not exceeding 8% by weight of the said aluminosilicate.

Zeolite MAP used to carry perfume and which is to be mixed with other constituents of a detergent composition may already be mixed with some other materials. Often these will be only a minority of the material present, until the zeolite MAP is mixed into the detergent composition. In one aspect this invention provides a particulate detergent composition containing from 2 to 60% by weight of detergent active and from 5 to 60% by weight of the alkali metal aluminosilicate of the zeolite P type having a silicon to aluminium ratio not greater than 1.33, and from 0.1 to 3% of perfume absorbed on the said aluminosilicate such that there is a greater concentration of perfume on said aluminosilicate than in the remainder of the composition, the weight of perfume being not more than 8% of the weight of the said aluminosilicate, with the proviso as described in claim 1.

When such a detergent composition is made, by mixing other ingredients with the aluminosilicate which has perfume absorbed thereby, the resulting composition will exhibit a greater concentration of perfume on the aluminosilicate than in the remainder of the composition.

In another aspect, this invention provides a method of making a particulate detergent composition which comprises
(i) absorbing perfume onto alkali metal aluminosilicate of the zeolite P type having a silicon to aluminium ratio not greater than 1.33,
(ii) subsequently, mixing the said aluminosilicate and absorbed perfume thereon with other ingredients of the composition including detergent active, the amount of said perfume being from 0.2 to 8% by weight of aluminosilicate of the said type in the detergent composition, with the proviso as described in claim 8.

In yet another aspect, this invention provides use of alkali metal aluminosilicate of the zeolite P type having a silicon to aluminium ratio not greater than 1.33 as a carrier material to incorporate perfume into a detergent composition and reduce the vapour pressure of the perfume, the amount of perfume on said aluminosilicate being not more than 8% by weight thereof.

### Further Description and Embodiments

As is well known, a perfume normally consists of a mixture of a number of perfumery materials, each of which has a fragrance. The number of perfumery materials in a perfume is typically ten or more. The range of fragrant materials used in perfumery is very wide; the materials come from a variety of chemical classes, but in general are hydrophobic oils. In many instances, the molecular weight of a perfumery material is in excess of 150, but does not exceed 300.

Although the invention is not limited to specific perfumery materials, some perfumery materials which may be used include: acetyl cedrene, 4-acetoxy-3-pentyltetrahydropyran, 4-acetyl-6-t-butyl-1,1-dimethylindane, available under the trademark "CELESTOLIDE",
5-acetyl-1,1,2,3,3,6-hexamethylindane, available under the trademark "PHANTOLIDE",
6-acetyl-1-isopropyl-2,3,3,5-tetramethylindane, available under the trademark "TRASEOLIDE",
alpha-n-amylcinammic aldehyde, amyl salicylate, aubepine, aubepine nitrile, aurantion,
2-t-butylcyclohexyl acetate, 2-t-butylcyclohexanol,
3 - (p-t-butylphenyl)propanal,
4-t-butylcyclohexyl acetate,
4-t-butyl-3,5-dinitro-2,6-dimethyl acetophenone,
4-t-butylcyclohexanol, benzoin siam resinoids,
benzyl benzoate, benzyl acetate,
benzyl propionate,
benzyl salicylate, benzyl isoamyl ether,
benzyl alcohol, bergamot oil, bornyl acetate,
butyl salicylate, carvacrol, cedar atlas oil,
cedryl methyl ether, cedryl acetate, cinnamic alcohol, cinnamyl propionate, cis-3-hexenol,
cis-3-hexenyl salicylate, citronella oil, citronellol,
citronellonitrile, citronellyl acetate,
citronellyloxyacetaldehyde, cloveleaf oil, coumarin,
9-decen-1-ol, n-decanal, n-dodecanal, decanol,
decyl acetate, diethyl phthalate, dihydromyrcenol,
dihydromyrcenyl formate, dihydromyrcenyl acetate,
dihydroterpinyl acetate,
dimethylbenzyl carbinyl acetate, dimethylbenzylcarbinol,
dimethylheptanol, dimethyloctanol, dimyrcetol, diphenyl oxide,
ethyl naphthyl ether, ethyl vanillin,
ethylene brassylate, eugenol, geraniol, geranium oil,
geranonitrile, geranyl nitrile, geranyl acetate,
1,1,2,4,4,7-hexamethyl-6-acetyl-1,2,3,4-tetrahydronaphthalene, available under the trademark "TONALID",
1,3,4,6,7,8-hexahydro-4,6,6,7,8,8-hexamethylcyclopenta-2-benzopyran, available under the trademark "GALAXOLIDE",
2-n-heptylcyclopentanone,
3a,4,5,6,7,7a-hexahydro-4,7-methano-1(3)H-inden-6-ylpropionate, available under the trademark "FLOROCYCLENE",
3a,4,5,6,7,7a-hexahydro-4,7-methano-1(3)H-inden-6-ylacetate, available under the trademark "JASMACYCLENE", 4-(4'-hydroxy-4'-methylpentyl)-3-cyclohexenecarbaldehyde, alpha-hexylcinammic aldehyde, heliotropin, Hercolyn D, hexyl aldone, hexyl cinnamic aldehyde,
hexyl salicylate, hydroxycitronellal, i-nonyl formate, 3-isocamphylcyclohexanol, 4-isopropylcyclohexanol, 4-isopropylcyclohexyl methanol, indole, ionones, irones, isoamyl salicylate, isoborneol,
isobornyl acetate, isobutyl salicylate, isobutylbenzoate, isobutylphenyl acetate, isoeugenol, isolongifolanone, isomethyl ionones, isononanol, isononyl acetate, isopulegol, lavendin oil,
lemongrass oil, linalool, linalyl acetate, LRG 201,
1-menthol, 2-methyl-3-(p-isopropylphenyl)propanal, 2-methyl-3-(p-t-butylphenyl)propanal,
3-methyl-2-pentyl-cyclopentanone, 3-methyl-5-phenyl-pentanol,
alpha and beta methyl naphthyl ketones, methyl ionones, methyl dihydrojasmonate, methyl naphthyl ether,
methyl 4-propyl phenyl ether, Mousse de chene Yugo,
Musk ambrette, myrtenol, neroli oil, nonanediol-1,3-diacetate, nonanol, nonanolide-1,4, nopol acetate,
1,2,3,4,5,6,7,8-octahydro-2,3,8,8-tetramethyl-2-acetyl-naphthalene, available under the trademark "ISO-E-SUPER",
octanol, Oppoponax resinoid, orange oil,
p-t-amylcyclohexanone,
p-t-butylmethylhydrocinnamic aldehyde,
2-phenylethanol, 2-phenylethyl acetate,
2-phenylpropanol, 3-phenylpropanol, para-menthan-7-ol, para-t-butylphenyl methyl ether, patchouli oil,
pelargene, petitgrain oil, phenoxyethyl isobutyrate, phenylacetaldehyde diethyl acetal,
phenylacetaldehyde dimethyl acetal,
phenylethyl n-butyl ether, phenylethyl isoamyl ether,
phenylethylphenyl acetate, pimento leaf oil,
rose-d-oxide, Sandalone, styrallyl acetate,
1,1,4,4-tetramethyl-6-acetyl-7-ethyl-1,2,3,4-tetrahydronaphthalene, available under the trademark "VERSALIDE",
3,3,5-trimethyl hexyl acetate,
3,5,5-trimethylcyclohexanol,
terpineol, terpinyl acetate, tetrahydrogeraniol, tetrahydrolinalool, tetrahydromuguol, tetrahydromyrcenol, thyme oil, trichloromethylphenycarbinyl acetate, tricyclodecenyl acetate, tricyclodecenyl propionate, 10-undecen-1-al, gamma undecalactone, 10-undecen-1-ol undecanol, vanillin, vetiverol, vetiveryl acetate, vetyvert oil, acetate and propionate esters of alcohols in the list above, aromatic nitromusk fragrances, indane musk fragrances, isochroman musk fragrances, macrocyclic ketones, macrolactone musk fragrances, and tetralin musk fragrances.

Perfumes frequently include solvents or diluents, for example: ethanol, isopropanol, diethylene glycol monoethyl ether, dipropylene glycol, diethyl phthalate and triethyl citrate.

Perfumes which are used in this invention may, if desired, have deodorant properties as disclosed in US-A-4303679, US-A-4663068 and EP-A-545556.

Forms of zeolite having the required properties to be useful in the present invention are disclosed in EP-A-384070 referred to above.

As stated in that document, such forms of zeolite are of the zeolite P type and have Si:Al ratios not greater than 1.33, i.e. the quantity of Si is not more than 1.33 times the quantity of Al. They have good calcium binding capacities, usually at least 150 mg CaO, preferably at least 160 mg CaO, per gram anhydrous aluminosilicate. The Si:Al ratio preferably lies within the range of from 0.9 to 1.33, more preferably from 0.9 to 1.2, most preferably from 0,9 : 1 to 1,15:1.

The aluminosilicates used in the present invention are in alkali metal salt form. The preferred cation is sodium.

It is desirable that the zeolite MAP has a small particle size. Desirably the average particle size is less than 4 micrometres. Desirably also, at least 90% by weight of the zeolite MAP has a particle size less than 7, better less than 5 micrometres.

A process for the preparation of zeolite MAP suitable for use in this invention is described in EP-A-565364.

The amount of perfume will generally be at least 0.5% by weight of the zeolite MAP. Up to at least 8% by weight of the zeolite, there is a trade off between the amount of perfume carried by the zeolite and the reduction in vapour pressure. In order to give more substantial reduction in vapour pressure it is preferred that the quantity of perfume is not more than 6%, better not more than 5% by weight of the zeolite. Preferably, the quantity of perfume is at least 1% and frequently at least 2% by weight of the zeolite.

The amount of perfume is typically 0,5 to 8%, preferably 1 to 8% and more preferably 0,5 to 6% by weight of said aluminosilicate.

Absorption of perfume onto the zeolite MAP can be carried out simply by mixing the perfume with the zeolite, possibly followed by a period of storage to allow equilibrium distribution to be obtained.

The amount of detergent active to be incorporated in a detergent composition of this invention lies in the broad range from 2% to 60% by weight.

Surfactants useful as detergent active in the detergent compositions herein include well-known anionic, nonionic, amphoteric and zwitterionic surfactants. Typical of these are the alkyl benzene sulphonates, alkyl sulphonates, allyl- and alkyl ether sulphates, primary alkyl sulphates, alkoxylated alcohols, alpha-sulphonates of fatty acids and of fatty acid esters, alkyl betaines, and polyalkyl glycosides all known in the detergent art.

Detergent active is preferably present in a quantity of at least 5% or 10% by weight of a composition, and may well be in a quantity not exceeding 50% or 40% by weight. Concentrated detergent compositions will generally contain detergent active in a quantity from 10% to 50% by weight of the composition.

The specified zeolite MAP provides from 5 to 60% of a detergent composition of this invention. The amount may be at least 10% and may lie in a range not exceeding 40% by weight. It may be the only zeolite present, or it may be used jointly with zeolite A. Generally if zeolite A is also present the total quantity of zeolite is not more than 80%, better 60% by weight of the composition.

The zeolite may be accompanied by a co-builder. Preferred are organic polymers containing carboxylate groups, especially co-polymers of maleate and acrylate. Such polymers may usefully be used in amounts from 1% to 10% by weight of the detergent composition.

Other detergency builders may also be present, notably the conventional inorganic and organic water soluble builder salts. Examples are alkali metal carbonates, borates, phosphates, polyphosphates, tripolyphosphates, bicarbonates, silicates, sulphates, and citric acid salts. The builder is preferably present at a total level not exceeding 80%, more preferably not over 60% by weight.

A detergent composition may include bleaches, notably peroxyacids such as diperoxydodecanedioic acid or phthaloylaminoperoxycaproic acid, or bleach systems that comprise a peroxide compound with liberates hydrogen peroxide in aqueous solution and an activator. Hydrogen peroxide sources are well known in the art, and include compounds such as alkali metal perborates, percarbonates, persulphates and persilicates, and urea peroxide; particularly percarbonate.

Preferred bleach activators are N,N,N,N-tetraacetylethylenediamine (TAED), 1,2,3,4,6-pentaacetylglucose (GPA), sodium-p-acetoxybenzenesulphonate (SABS), sodium-p-benzoyloxybenzenesulphonate (SBOBS), sodium-p-nonanoyl-oxybenzenesulphonate (SNOBS), sodium-p-3,5,5-trimethylhexanoyloxy-benzenesulphonate (iso-SNOBS), 2-N,N,N-trimethylammonioethyl-4-sulphophenylcarbonate (CSPC), and transition metal catalysts disclosed in EP-A-458397, EP-A-458398 and EP-A-549272.

Other ingredients which may optionally be present in a detergent composition include fabric softening agents such as fatty amines, fabric softening clay materials, lather depressants, and, usually present in very minor amounts, fluorescent agents, perfumes including deodorant perfumes, enzymes such as proteases, cellulases, amylases and lipase, germicides and colorants.

Detergent compositions of this invention will generally be in solid form, e.g. granular products commonly referred to as powders.

Preferably, a detergent composition is made by the method which is an aspect of this invention in which the perfume is absorbed on zeolite MAP, after which this zeolite MAP is mixed with other ingredients of the composition.

In this procedure, it is possible to mix perfume with only part of the zeolite MAP which is present in the final composition. If so, the perfume absorbed on zeolite MAP might, for a time, exceed 8% of the weight of zeolite which is carrying it. When this zeolite MAP is mixed into the rest of the composition including more zeolite MAP, the proportion of perfume relative to the overall quantity of zeolite MAP will of course then fall. The proportions should be arranged so that the amount of perfume is not over 8% by weight of the total quantity of zeolite MAP.

It is also possible to obtain the reduction in vapour pressure of perfume even when the perfume is added to a detergent composition which has already been prepared from its constituted materials.

### Examples

### Example 1

Zeolite MAP was produced as described in EP-A-565364. Its micropore volume, that is to say pores in the approximate range 7 to 50 angstroms, was measured by the nitrogen absorption methods referred to in EP-A-535942, and found to be less than 0.02 ml/g.

Its volume of pores of larger size was then determined by mercury porosimetry and found to be 0.9 ml/g.

Particle size was determined using a Malvern Mastersizer instrument. Average size was found to be 0.9 *µ*m with about 60% of particles in the range 0.6 to 1 *µ*m.

A number of samples of this zeolite MAP were used to absorb varying concentrations of a perfume composition intended for the perfuming of fabric washing detergent products. To absorb the perfume, a weighed quantity of perfume was poured onto a weighed quantity of the zeolite MAP in a glass screw top bottle. The contents were then mixed by stirring with a glass rod or stainless steel spatula until the mixture showed no signs of free liquid or "wet" lumps. More zeolite was then added with constant stirring until the required perfume to zeolite ratio was reached. The bottle was then sealed and roller mixed for several days to equilibrate the sample. Occasionally during this time any solid lumps were broken up by manual stirring.

The quantity of perfume in the headspace over the zeolite was determined by gas chromatographic headspace analysis. A 1 g sample of the zeolite was placed in a 20 ml crimped top vial, and allowed to equilibrate for one hour at 60°C. The headspace was then analysed using a Perkin Elmer HS 101 headspace auto-sampler linked to a Perkin Elmer 8500 gas chromatograph. The column used was 2 metres of Carbowax 20M (polyethylene glycol, average MW 15,000-20,000) on Chromosorb W.HP (white diatomaceous earth) available from Hewlett Packard. Results are quoted as the total peak area.

Samples prepared and analysed in this way contained 2%, 5%, 10% and 20% of perfume by weight of zeolite MAP. A comparison was carried out using zeolite 4A. The results obtained were:

| Perfume concentrations | Peak area zeolite MAP | Peak area zeolite 4A |
|---|---|---|
| 2% | 45 | 190 |
| 5% | 140 | |
| 10% | 220 | 230 |
| 20% | 220 | |

These results show that 10% perfume saturates the zeolite MAP, so that there is no vapour pressure reduction. At 2% and 5% however, the vapour pressure of perfume was 20% and 64% of the vapour pressure at saturation. By context, zeolite 4A was almost saturated at 2% concentration. Thus at concentrations below 10%, the zeolite MAP is much more effective than zeolite 4A in reducing perfume vapour pressure.

### Example 2

Samples of the two zeolites, each carrying 2% perfume, were added to equal values of distilled water. The perfume in the head space was then measured by gas chromatography as in the previous example.

There was very little difference in the peak areas, indicating complete release of the perfume on contact with water.

### Example 3

The above results were confirmed by a panel test. 0.5 g of zeolite MAP or of zeolite 4A, carrying in either case absorbed perfume at a concentration of 2% by weight based on the zeolite, were placed in 50 ml screw top jars and allowed to equilibrate for 1 hour at 27°C.

Two jars, one of each were then presented to each of 11 panellists who were asked to decide which contained the stronger perfume. This experiment was repeated twice, The panellists unanimously picked the jars containing zeolite MAP as having the weaker perfume, thus indicating the greater ability of zeolite MAP to absorb and retain perfume.

### Example 4

A quantity of zeolite MAP was divided into two portions. One was made to absorb 2% perfume, as in Example 1.

0.70g samples of each zeolite were weighed into separate 500ml aliquots of a solution containing 0.01M sodium chloride and 3.92 x 10⁻³M calcium chloride, maintained at 25°C. The calcium ion exchange capacity was determined after 30 minutes by immersing a calcium sensitive electrode in the solution.

The values obtained were 159 mg/gram for zeolite which did not carry perfume and 157 mg/gram for the zeolite which did carry perfume. The difference in these values was not significant. It was thus confirmed that zeolite MAP could be used as a perfume carrier in a detergent composition and then function as a detergency builder when the composition is added to water at the time of use.

### Example 5

Two detergent compositions were prepared containing:

| | parts by weight |
|---|---|
| coconut alkyl sulphate | 8 |
| nonionic surfactant (C₁₃ alcohol 7EO) | 9 |
| zeolite | 35 |
| sodium carbonate | 10 |
| sodium stearate 2 | |
| acrylic maleic copolymer (Sokolan CP5) | 2 |
| sodium carboxymethyl cellulose | 1 |
| perfume | 0.7 |

[These compositions were models for a product containing 8% anionic detergent, 9% nonionic, 35% zeolite and 0.7% perfume, and including a bleach system]

In one composition the zeolite was zeolite 4A in the other it was zeolite MAP. In both cases the perfume was 2% of the weight of zeolite and was absorbed on the zeolite, as in Example 1, before adding the zeolite to the remainder of the composition.

Gas chromatography was used to measure the concentration of perfume in the headspace over the detergent composition, using the same procedure as in Example 1.

It was found that the intensity of the perfume over the composition containing zeolite 4A was 10% greater than over the composition containing zeolite MAP, indicating that zeolite MAP retains its ability to absorb and retain perfume even when incorporated into a detergent composition with nonionic detergent present.

The intensity of perfume in the headspace over the two compositions was also assessed by a panel, with a procedure as in Example 3, placing 3g of one or other composition in the 50ml screw topped jars. A total of 99 assessments was made by the panel who decided by a ratio of 2:1 that the perfume over the composition containing zeolite MAP was weaker.

### Example 6

A perfume containing more than 10 fragrance materials was absorbed onto zeolite MAP by the procedure of Example 1. Samples of the mixture were then stored in sealed jars at room temperature for 3 months.

After this time the perfume remaining in the composition was extracted with solvent and analysed by gas chromatography. It was found that 68% of the original quantity of perfume remained and that chromatography peaks corresponding to many of the original fragrance materials were still present.

### Example 7

A granular detergent composition is prepared to the following formulation:

| | % |
|---|---|
| Linear alkylbenzene sulphonate | 25.0 |
| Nonionic Surfactant | 1.5 |
| Soap | 1.0 |
| Zeolite MAP (anhydrous) | 35.0 |
| Perfume | 1.0 |
| Sodium silicate | 4.0 |
| Acrylic maleic copolymer | 1.5 |
| Fluorescer | 0.18 |
| Sodium carboxymethyl cellulose | 0.6 |
| Sodium Carbonate | 14.3 |
| Enzyme (Alcalase (Trade Mark)) | 0.6 |
| Antifoam | 0.04 |
| Alusil N (finely divided silica flow aid) | 2.5 |
| Other minor ingredients | balance to 100% |

The perfume is carried on the zeolite MAP, and is thus present at a concentration of approximately 3% by weight based on the zeolite.

The composition is prepared by absorbing all the perfume on one quarter (or alternatively one half) the zeolite MAP, and mixing this into the remainder of the detergent composition - which may itself be prepared by mixing the ingredients or else by spray drying those ingredients which are not sensitive to heat, and mixing in the others.

### Example 8

A granular detergent composition is prepared to the following formulation:

| | % |
|---|---|
| Linear alkylbenzene sulphonate | 6.6 |
| Tallow alkyl sulphate | 2.4 |
| Nonionic surfactant (C₁₃ alcohol 7EO) | 6.1 |
| Zeolite MAP (anhydrous) | 22 |
| Perfume | 0.8 |
| Sodium silicate | 2.7 |
| Polyacrylate | 5.6 |
| Sodium perborate tetrahydrate | 14.0 |
| Tetraacetyl ethylene diamine (TAED) | 6.1 |
| Fluorescer | 0.5 |
| Sodium carboxymethyl cellulose | 0.5 |
| Sodium carbonate | 17.3 |
| Enzyme (Savinase (Trade Mark)) | 1.6 |
| Silicone antifoam | 0.16 |
| Sodium sulphate, water and other minor ingredients | balance to 100% |

The perfume is carried on the zeolite MAP, and is thus again present at a concentration of approximately 3% by weight based on the zeolite. This composition can be prepared in the same way as for the preceding example.

## Claims (Claims for the following Contracting State(s): DE, FR, GB, IT, NL)

1. A particulate detergent composition comprising:
2 to 60% by weight of detergent active,
5 to 60% by weight of alkali metal aluminosilicate of the zeolite P type having a silicon to aluminium ratio not greater than 1.33, and
0.1 to 3% by weight perfume which is at least partly absorbed on the said aluminosilicate, such that there is a greater concentration of perfume on said aluminosilicate than in the remainder of the composition the weight of perfume being not more than 8% of the weight of the said aluminosilicate;
with the proviso that the particulate detergent composition is other than a composition of density 700 gm/l containing said aluminosilicate of the zeolite P type :18%
co-polymer of 1:4 maleic/acrylic acids: 4%
diethylene triamine penta (methylene phosphonic acid): 0.5%
brightener: 0.2%
magnesium sulphate: 0.4%
perfume: 0.1%
C₁₄ - C₁₅ alcohol ethoxylate with average 7 ethoxy groups per mole of alcohol: 4%
silicone suds suppressor: 0.5%
tallow alcohol having average 11 ethylene oxide groups per mole of alcohol: 1%
percarbonate bleach: 18%
tetraacetyl ethylene diamine: 5%
trisodium citrate dihydrate: 10%
linear C₈₋₁₁ alkylbenzene sulphonate: 7%
sodium C_{16 - 18} alkyl sulphate: 2%
C_{12 - 15} alcohol ethoxysulphate having an average of 3 ethoxy groups per mole: 0.2%
sodium carboxymethyl cellulose: 0.3%
sodium carbonate: 15%
Sodium bicarbonate: 2%
additional perfume as 40% perfume in dextrin capsules: 0.2%
Savinase: 1.5%
other ingredients to a balance of 100%

2. A particulate detergent composition according to claim 1 wherein the amount of perfume is from 0.5 to 8% by weight of the said aluminosilicate.

3. A particulate composition according to claim 1 or claim 2 wherein the amount of perfume is from 1 to 8% by weight of the said aluminosilicate.

4. A particulate composition according to claim 1 or claim 2 wherein the amount of perfume is from 0.5 to 6% by weight of the said aluminosilicate.

5. A particulate composition according to claim 1 wherein the ratio of Si to Al in the said aluminosilicate lies in a range from 0.9:1 to 1.15:1.

6. A particulate composition according to any one of the preceding claims wherein the perfume comprises perfumery material with a molecular weight which exceeds 150 but does not exceed 300.

7. A composition according to any one of the preceding claims which contains from 10 to 40% by weight of the said aluminosilicate.

8. A method of making a particulate detergent composition comprising 2 to 60% by weight of detergent active, 5 to 60% by weight of alkali metal aluminosilicate of the zeolite P type having a silicon to aluminium ratio not greater than 1.33, and 0.1 to 3% by weight perfume, which method comprises
i) absorbing perfume onto alkali metal aluminosilicate of the zeolite P type having a silicon to aluminium ratio not greater than 1.33,
ii) mixing the said aluminosilicate and absorbed perfume thereon with other ingredients of the composition including detergent active, the amount of said perfume being from 0.2 to 8% by weight of aluminosilicate of the said type in the detergent composition;
with the proviso that the resulting detergent composition is other than a composition of density 700 gm/1 containing said aluminosilicate of the zeolite P type :18%
co-polymer of 1:4 maleic/acrylic acids: 4%
diethylene triamine penta (methylene phosphonic acid): 0.5%
brightener: 0.2%
magnesium sulphate: 0.4%
perfume: 0.1%
C₁₄ - C₁₅ alcohol ethoxylate with average 7 ethoxy groups per mole of alcohol: 4%
silicone suds suppressor: 0.5%
tallow alcohol having average 11 ethylene oxide groups per mole of alcohol: 1%
percarbonate bleach: 18%
tetraacetyl ethylene diamine: 5%
trisodium citrate dihydrate: 10%
linear C₈₋₁₁ alkylbenzene sulphonate: 7%
sodium C_{16 - 18} alkyl sulphate: 2%
C_{12 - 15} alcohol ethoxysulphate having an average of 3 ethoxy groups per mole: 0.2%
sodium carboxymethyl cellulose: 0.3%
sodium carbonate: 15%
Sodium bicarbonate: 2%
additional perfume as 40% perfume in dextrin capsules: 0.2% Savinase : 1.5%
other ingredients to a balance of 100%

9. A method according to claim 8 wherein the amount of perfume is from 0.5 to 8% by weight of the said aluminosilicate.

10. A method according to claim 8 wherein the amount of perfume is from 1 to 8% by weight of the said aluminosilicate.

11. A method according to claim 8 wherein the amount of perfume is from 0.5 to 6% by weight of the said aluminosilicate.

12. A method according to any one of claims 8 to 11 wherein the perfume comprises perfumery material with a molecular weight which exceeds 150 but does not exceed 300.

13. A method according to any one of claims 8 to 12 wherein the amount of the said aluminosilicate is from 10 to 40% by weight of the composition.

14. A method according to any one of claims 8 to 13 wherein the ratio of Si to Al in the said aluminosilicate lies in a range from 0.9:1 to 1.15:1.

15. Use of alkali metal aluminosilicate of the zeolite P type having a silicon to aluminium ratio not greater than 1.33 as a carrier material to incorporate perfume into a detergent composition and reduce the vapour pressure of the perfume, the amount of perfume on said aluminosilicate being not more than 8% by weight thereof.

## Claims (Claims for the following Contracting State(s): ES)

1. A particulate detergent composition comprising:
2 to 60% by weight of detergent active,
5 to 60% by weight of alkali metal aluminosilicate of the zeolite P type having a silicon to aluminium ratio not greater than 1.33, and
0.1 to 3% by weight perfume which is at least partly absorbed on the said aluminosilicate, such that there is a greater concentration of perfume on said aluminosilicate than in the remainder of the composition, the weight of perfume being not more than 8% of the weight of the said aluminosilicate.

2. A particulate detergent composition according to claim 1 wherein the amount of perfume is from 0.5 to 8% by weight of the said aluminosilicate.

3. A particulate composition according to claim 1 wherein the amount of perfume is from 1 to 8% by weight of the said aluminosilicate.

4. A particulate composition according to claim 1 wherein the amount of perfume is from 0.5 to 6% by weight of the said aluminosilicate.

5. A particulate composition according to any one of claims 1 to 4 wherein the ratio of Si to Al in the said aluminosilicate lies in a range from 0.9:1 to 1.15:1.

6. A particulate composition according to any one of the preceding claims wherein the perfume comprises perfumery material with a molecular weight which exceeds 150 but does not exceed 300.

7. A composition according to any one of the preceding claims which contains from 10 to 40% by weight of the said aluminosilicate.

8. A method of making a particulate detergent composition comprising 2 to 60% by weight of detergent active, 5 to 60% by weight of alkali metal aluminosilicate of the zeolite P type having a silicon to aluminium ratio not greater than 1.33, and 0.1 to 3% by weight perfume, which method comprises
i) absorbing perfume onto alkali metal aluminosilicate of the zeolite P type having a silicon to aluminium ratio not greater than 1.33,
ii) mixing the said aluminosilicate and absorbed perfume thereon with other ingredients of the composition including detergent active, the amount of said perfume being from 0.2 to 8% by weight of aluminosilicate of the said type in the detergent composition.

9. A method according to claim 8 wherein the amount of perfume is from 0.5 to 8% by weight of the said aluminosilicate.

10. A method according to claim 8 wherein the amount of perfume is from 1 to 8% by weight of the said aluminosilicate.

11. A method according to claim 8 wherein the amount of perfume is from 0.5 to 6% by weight of the said aluminosilicate.

12. A method according to any one of claims 8 to 11 wherein the perfume comprises perfumery material with a molecular weight which exceeds 150 but does not exceed 300.

13. A method according to any one of claims 8 to 12 wherein the amount of the said aluminosilicate is fro 10 to 40% by weight of the composition.

14. A method according to any one of claims 8 to 13 wherein the ratio of Si to Al in the said aluminosilicate lies in a range from 0.9:1 to 1.15:1.

15. Use of alkali metal aluminosilicate of the zeolite P type having a silicon to aluminium ratio not greater than 1.33 as a carrier material to incorporate perfume into a detergent composition and reduce the vapour pressure of the perfume, the amount of perfume on said aluminosilicate being not more than 8% by weight thereof.

## Patentansprüche (Patentansprüche für folgende(n) Vertragsstaat(en): DE, FR, GB, IT, NL)

1. Eine teilchenförmige Detergens-Zusammensetzung, enthaltend:
2 bis 60 Gew.-% des aktiven Detergens,
5 bis 60 Gew.-% des Alkalimetallaluminosilicats vom Zeolith P-Typ mit einem Silicium- zu Aluminium-Verhältnis von nicht größer als 1,33, und
0,1 bis 3 Gew.-% Parfüm, welches zumindest teilweise auf dem erwähnten Aluminosilicat absorbiert ist, derart, daß darin eine größere Konzentration Parfüm auf dem erwähnten Aluminosilicat ist, als in dem Rest der Zusammensetzung, wobei das Gewicht des Parfüms nicht mehr als 8 % des Gewichts des erwähnten Aluminosilicats ist;
mit der Maßgabe, daß die teilchenförmige Detergens-Zusammensetzung anders ist als eine Zusammensetzung der Dichte von 700 g/ℓ, enthaltend das erwähnte Aluminosilicat des Zeolith P-Typs: 18 % Copolymeres von 1 : 4 Malein-/Acrylsäuren: 4 %
Diethylentriamin-penta(methylenphosphonsäure): 0,5 %
Abklärer: 0,2 %
Magnesiumsulfat: 0,4 %
Parfüm: 0,1 %
C₁₄₋₁₅-Alkoholethoxylat mit durchschnittlich 7 Ethoxy-Gruppen pro Mol Alkohol: 4 %
Silicon-Schaumunterdrücker: 0,5 %
Talgalkohol mit einem Durchschnitt von 11 Ethylenoxid-Gruppen pro Mol Alkohol: 1 %
Percarbonat-Bleichmittel: 18 %
Tetraacetylethylendiamin: 5 %
Trinatriumcitrat.Dihydrat: 10 %
Lineares C₈₋₁₁-Alkylbenzolsulfonat: 7 %
Natrium-C₁₆₋₁₈-alkylsulfat: 2 %
C₁₂₋₁₅-Alkoholethoxysulfat mit einem Durchschnitt von 3 Ethoxy-Gruppen pro Mol: 0,2 %
Natriumcarboxymethylcellulose: 0,3 %
Natriumcarbonat: 15 %
Natriumbicarbonat: 2 %
Zusätzliches Parfüm als 40 % Parfüm in Dextrin-Kapseln: 0,2 %
Savinase: 1,5 %
Andere Bestandteile bis zu einem Rest von 100 %.

2. Eine teilchenförmige Detergens-Zusammensetzung nach Anspruch 1, worin die Menge des Parfüms von 0,5 bis 8 Gew.-% des erwähnten Aluminosilicats beträgt.

3. Eine teilchenförmige Zusammensetzung nach Anspruch 1 oder Anspruch 2, worin die Menge des Parfüms von 1 bis 8 Gew.-% des erwähnten Aluminosilicats beträgt.

4. Eine teilchenförmige Zusammensetzung nach Anspruch 1 oder Anspruch 2, worin die Menge des Parfüms von 0,5 bis 6 Gew.-% des erwähnten Aluminosilicats beträgt.

5. Eine teilchenförmige Zusammensetzung nach Anspruch 1, worin das Verhältnis von Si zu Al in dem erwähnten Aluminosilicat in einem Bereich von 0,9 : 1 bis 1,15 : 1 liegt.

6. Eine teilchenförmige Zusammensetzung nach irgendeinem der vorstehenden Ansprüche, worin das Parfüm Parfümerie-Material mit einem Molekulargewicht umfaßt, welches 150 übersteigt, jedoch 300 nicht übersteigt.

7. Eine Zusammensetzung nach irgendeinem der vorstehenden Ansprüche, welche von 10 bis 40 Gew.-% des erwähnten Aluminosilicats enthält.

8. Ein Verfahren zur Herstellung einer teilchenförmigen Detergens-Zusammensetzung, enthaltend 2 bis 60 Gew.-% aktives Detergens, 5 bis 60 Gew.-% Alkalimetallaluminosilicat des Zeolith P-Typs mit einem Silicium- zu Aluminium-Verhältnis von nicht größer als 1,33, und 0,1 bis 3 Gew.-% Parfüm, welches Verfahren umfaßt
(i) das Absorbieren von Parfüm auf Alkalimetallaluminosilicat des Zeolith P-Typs mit einem Silicium- zu Aluminium-Verhältnis von nicht größer als 1,33,
(ii) Mischen des erwähnten Aluminosilicats und des absorbierten Parfüms darauf mit anderen Bestandteilen der Zusammensetzung, einschließend aktives Detergens, wobei die Menge des erwähnten Parfüms im Bereich von 0,2 bis 8 Gew.-% des Aluminosilicats des erwähnten Typs in der Detergens-Zusammensetzung ist;
mit der Maßgabe, daß die resultierende Detergens-Zusammensetzung anders ist als eine Zusammensetzung der Dichte von 700 g/ℓ, enthaltend das erwähnte Aluminosilicat des Zeolith P-Typs: 18 %
Copolymeres von 1 : 4 Malein-/Acrylsäuren: 4 %
Diethylentriamin-penta(methylenphosphonsäure): 0,5 %
Abklärer: 0,2 %
Magnesiumsulfat: 0,4 %
Parfüm: 0,1 %
C₁₄₋₁₅-Alkoholethoxylat mit durchschnittlich 7 Ethoxy-Gruppen pro Mol Alkohol: 4 %
Silicon-Schaumunterdrücker: 0,5 %
Talgalkohol mit einem Durchschnitt von 11 Ethylenoxid-Gruppen pro Mol Alkohol: 1 %
Percarbonat-Bleichmittel: 18 %
Tetraacetylethylendiamin: 5 %
Trinatriumcitrat.Dihydrat: 10 %
Lineares C₈₋₁₁-Alkylbenzolsulfonat: 8 %
Natrium-C₁₆₋₁₈-alkylsulfat: 2 %
C₁₂₋₁₅-Alkoholethoxysulfat mit einem Durchschnitt von 3 Ethoxy-Gruppen pro Mol: 0,2 %
Natriumcarboxymethylcellulose: 0,3 %
Natriumcarbonat: 15 %
Natriumbicarbonat: 2 %
Zusätzliches Parfüm als 40 % Parfüm in Dextrin-Kapseln: 0,2 %
Savinase: 1,5 %
Andere Bestandteile bis zu einem Rest von 100 %.

9. Ein Verfahren nach Anspruch 8, worin die Menge des Parfüms von 0,5 bis 8 Gew.-% des erwähnten Aluminosilicats beträgt.

10. Ein Verfahren nach Anspruch 8, worin die Menge des Parfüms von 1 bis 8 Gew.-% des erwähnten Aluminosilicats beträgt.

11. Ein Verfahren nach Anspruch 8, worin die Menge des Parfüms von 0,5 bis 6 Gew.-% des erwähnten Aluminosilicats beträgt.

12. Ein Verfahren nach irgendeinem der Ansprüche 8 bis 11, worin das Parfüm Parfümerie-Material mit einem Molekulargewicht umfaßt, welches 150 übersteigt, jedoch 300 nicht übersteigt.

13. Ein Verfahren nach irgendeinem der Ansprüche 8 bis 12, worin die Menge des erwähnten Aluminosilicats von 10 bis 40 Gew.-% der Zusammensetzung beträgt.

14. Ein Verfahren nach irgendeinem der Ansprüche 8 bis 13, worin das Verhältnis von Si zu Al in dem erwähnten Aluminosilicat in einem Bereich von 0,9 : 1 bis 1,15 : 1 liegt.

15. Die Verwendung des Alkalimetallaluminosilicats des Zeolith P-Typs mit einem Silicium- zu Aluminium-Verhältnis von nicht größer als 1,33 als ein Trägermaterial zur Inkorporierung von Parfüm in eine Detergens-Zusammensetzung und Reduzieren des Dampfdrucks des Parfüms, wobei die Menge des Parfüms auf dem erwähnten Aluminosilicat nicht mehr als 8 Gew.-% davon beträgt.

## Patentansprüche (Patentansprüche für folgende(n) Vertragsstaat(en): ES)

1. Eine teilchenförmige Detergens-Zusammensetzung, enthaltend:
2 bis 60 Gew.-% des aktiven Detergens,
5 bis 60 Gew.-% des Alkalimetallaluminosilicats vom Zeolith P-Typ mit einem Silicium- zu Aluminium-Verhältnis von nicht größer als 1,33, und
0,1 bis 3 Gew.-% Parfüm, welches zumindest teilweise auf dem erwähnten Aluminosilicat absorbiert ist, derart, daß darin eine größere Konzentration Parfüm auf dem erwähnten Aluminosilicat ist, als in dem Rest der Zusammensetzung, wobei das Gewicht des Parfüms nicht mehr als 8 % des Gewichts des erwähnten Aluminosilicats ist.

2. Eine teilchenförmige Detergens-Zusammensetzung nach Anspruch 1, worin die Menge des Parfüms von 0,5 bis 8 Gew.-% des erwähnten Aluminosilicats beträgt.

3. Eine teilchenförmige Zusammensetzung nach Anspruch 1, worin die Menge des Parfüms von 1 bis 8 Gew.-% des erwähnten Aluminosilicats beträgt.

4. Eine teilchenförmige Zusammensetzung nach Anspruch 1, worin die Menge des Parfüms von 0,5 bis 6 Gew.-% des erwähnten Aluminosilicats beträgt.

5. Eine teilchenförmige Zusammensetzung nach irgendeinem der Ansprüche 1 bis 4, worin das Verhältnis von Si zu Al in dem erwähnten Aluminosilicat im Bereich von 0,9 : 1 bis 1,15 : 1 liegt.

6. Eine teilchenförmige Zusammensetzung nach irgendeinem der vorstehenden Ansprüche, worin das Parfüm Parfümerie-Material mit einem Molekulargewicht umfaßt, welches 150 übersteigt, jedoch 300 nicht übersteigt.

7. Eine Zusammensetzung nach irgendeinem der vorstehenden Ansprüche, welche von 10 bis 40 Gew.-% des erwähnten Aluminosilicats enthält.

8. Ein Verfahren zur Herstellung einer teilchenförmigen Detergens-Zusammensetzung, enthaltend 2 bis 60 Gew.-% aktives Detergens, 5 bis 60 Gew.-% Alkalimetallaluminosilicat des Zeolith P-Typs mit einem Silicium- zu Aluminium-Verhältnis von nicht größer als 1,33 und 0,1 bis 3 Gew.-% Parfüm, welches Verfahren umfaßt
(i) das Absorbieren von Parfüm auf Alkalimetallaluminosilicat des Zeolith P-Typs mit einem Silicium- zu Aluminium-Verhältnis von nicht größer als 1,33,
(ii) Mischen des erwähnten Aluminosilicats und des absorbierten Parfüms darauf mit anderen Bestandteilen der Zusammensetzung einschließend aktives Detergens, wobei die Menge des erwähnten Parfüms im Bereich von 0,2 bis 8 Gew.-% des Aluminosilicats des erwähnten Typs in der Detergens-Zusammensetzung ist.

9. Ein Verfahren nach Anspruch 8, worin die Menge des Parfüms von 0,5 bis 8 Gew.-% des erwähnten Aluminosilicats beträgt.

10. Ein Verfahren nach Anspruch 8, worin die Menge des Parfüms von 1 bis 8 Gew.-% des erwähnten Aluminosilicats beträgt.

11. Ein Verfahren nach Anspruch 8, worin die Menge des Parfüms von 0,5 bis 6 Gew.-% des erwähnten Aluminosilicats beträgt.

12. Ein Verfahren nach irgendeinem der Ansprüche 8 bis 11, worin das Parfüm Parfümerie-Material mit einem Molekulargewicht umfaßt, welches 150 übersteigt, jedoch 300 nicht übersteigt.

13. Ein Verfahren nach irgendeinem der Ansprüche 8 bis 12, worin die Menge des erwähnten Aluminosilicats von 10 bis 40 Gew.-% der Zusammensetzung beträgt.

14. Ein Verfahren nach irgendeinem der Ansprüche 8 bis 13, worin das Verhältnis von Si zu Al in dem erwähnten Aluminosilicat in einem Bereich von 0,9 : 1 bis 1,15 : 1 liegt.

15. Die Verwendung des Alkalimetallaluminosilicats des Zeolith P-Typs mit einem Silicium- zu Aluminium-Verhältnis von nicht größer als 1,33 als ein Trägermaterial zur Inkorporierung von Parfüm in eine Detergens-Zusammensetzung und Reduzieren des Dampfdrucks des Parfüms, wobei die Menge des Parfüms auf dem erwähnten Aluminosilicat nicht mehr als 8 Gew.-% davon beträgt.

## Revendications (Revendications pour l'(les) Etat(s) contractant(s) suivant(s): DE, FR, GB, IT, NL)

1. Composition détergente sous forme de particules contenant :
de 2 à 60 % en masse d'un ingrédient détergent actif ;
de 5 à 60 % en masse d'un aluminosilicate de métal alcalin du type zéolite P
ayant un rapport silicium sur aluminium qui n'est pas supérieur à 1,33, et
de 0,1 à 3 % en masse de parfum qui est au moins partiellement absorbé sur ledit aluminosilicate, de telle sorte qu'il y ait une concentration de parfum plus importante sur ledit aluminosilicate que dans le reste de la composition, la
masse de parfum n'étant pas supérieure à 8 % en masse dudit aluminosilicate ;
sous réserve que la composition détergente sous forme de particules soit différente d'une composition d'une densité de 700 grammes/litre et contienne :
ledit aluminosilicate du type zéolite P : 18 %
du copolymère d'acides maléique/acrylique dans une proportion de 1 pour 4 : 4 %
acide diéthylène triamine penta (méthylène phosphonique) :0,5 %
Eclaircissant : 0,2 %
sulfate de magnésium : 0,4 %
parfum : 0,1 %
éthoxylat d'alcool en C₁₄ - C₁₅, avec en moyenne 7 groupes éthoxy par mole d'alcool : 4 %
supresseurs de mousse silicone : 0,5 %
alcool de suif ayant en moyenne 11 groupes d'oxyde d'éthylène par mole
d'alcool : 1 %
agent blanchissant percarbonate : 18 %
tétraacétyl éthylène diamine : 5 %
citrate de trisodium dihydraté : : 10 %
alkyl benzène sulfonate linéaire en C₈ - C₁₁ : 7 %
alkyl sulfate de sodium en C₁₆ - C₁₈ : 2 %
éthoxysulfate d'alcool en C₁₂ - C₁₅ ayant en moyenne 3 groupes éthoxy par mole : 0,2 %
carboxyméthyl cellulose de sodium : 0,3 %
carbonate de sodium : 15 %
bicarbonate de sodium : 2 %
parfum additionnel sous la forme de 40 % de parfum dans des capsules de dextrine : 0,2 %
Savinase : 1,5 %
autres ingrédients : qsp 100

2. Composition détergente sous forme de particules selon la revendication 1, dans laquelle la quantité de parfum va de 0,5 à 8 % en masse dudit aluminosilicate.

3. Composition sous forme de particules selon la revendication 1 ou 2, dans laquelle la quantité de parfum va de 1 à 8 % en masse dudit aluminosilicate.

4. Composition sous forme de particules selon la revendication 1 ou 2, dans laquelle la quantité de parfum va de 0,5 à 6 % en masse dudit aluminosilicate.

5. Composition sous forme de particules selon la revendication 1, dans laquelle le rapport entre Si et Al dans ledit aluminosilicate est compris dans la gamme allant de 0,9 pour 1 à 1,15 pour 1.

6. Composition sous forme de particules selon l'une des revendications précédentes, dans laquelle le parfum comprend des matériaux de parfumerie ayant une masse moléculaire supérieure à 150 mais ne dépassant pas 300.

7. Composition selon l'une des revendications précédentes, contenant de 10 à 40 % en masse dudit aluminosilicate.

8. Procédé de fabrication d'une composition détergente sous forme de particules comprenant de 2 à 60 % en masse d'ingrédient détergent actif, de 5 à 60 % en masse d'aluminosilicate de métal alcalin du type zéolite P ayant un rapport silicium sur aluminium qui n'est pas supérieur à 1,33, et de 0,1 à 3 % en masse de parfum, ledit procédé comprenant les étapes consistant à :
(i) absorber le parfum sur un aluminosilicate de métal alcalin du type zéolite P ayant un rapport silicium/aluminium qui n'est pas supérieur à 1,33 ;
(ii) ensuite, mélanger ledit aluminosilicate et le parfum absorbé dessus avec les autres ingrédients de la composition comprenant le matériau détergent actif, la quantité dudit parfum étant comprise entre 0,2 et 8 % en masse de l'aluminosilicate dudit type dans la composition ;
sous réserve que la composition détergente sous forme de particules qui en résulte soit différente d'une composition d'une densité de 700 grammes/litre et contienne :
ledit aluminosilicate du type zéolite P : 18 %
du copolymère d'acides maléique/acrylique dans une proportion de 1 pour 4 : 4 %
acide diéthylène triamine penta (méthylène phosphonique) :0,5 %
éclaircissant : 0,2 %
sulfate de magnésium : 0,4 %
parfum: 0,1 %
éthoxylat d'alcool en C₁₄ - C₁₅, avec en moyenne 7 groupes éthoxy par mole d'alcool : 4 %
supresseurs de mousse silicone : 0,5 %
alcool de suif ayant en moyenne 11 groupes d'oxyde d'éthylène par mole d'alcool : 1 %
agent blanchissant percarbonate : 18 %
tétraacétyl éthylène diamine : 5 %
citrate de trisodium dihydraté : : 10 %
alkyl benzène sulfonate linéaire en C₈ - C₁₁ : 7 %
alkyl sulfate de sodium en C₁₆ - C₁₈ : 2 %
éthoxysulfate d'alcool en C₁₂ - C₁₅ ayant en moyenne 3 groupes éthoxy par mole : 0,2 %
carboxyméthyl cellulose de sodium : 0,3 %
carbonate de sodium : 15 %
bicarbonate de sodium : 2 %
parfum additionnel sous la forme de 40 % de parfum dans des capsules de dextrine : 0,2 %
Savinase : 1,5 %
autres ingrédients : qsp 100

9. Procédé selon la revendication 8, dans lequel la quantité de parfum va de 0,5 à 8 % en masse dudit aluminosilicate.

10. Procédé selon la revendication 8, dans lequel la quantité de parfum va de 1 à 8 % en masse dudit aluminosilicate.

11. Procédé selon la revendication 8, dans lequel la quantité de parfum va de 0,5 à 6 % en masse dudit aluminosilicate.

12. Procédé selon l'une des revendications 8 à 11 dans lequel le parfum comprend un matériau de parfumerie ayant une masse moléculaire supérieure à 150 mais ne dépassant pas 300.

13. Procédé selon l'une des revendications 8 à 12, dans lequel la quantité dudit aluminosilicate va de 10 à 40 % en masse de la composition.

14. Procédé selon l'une des revendications 8 à 13, dans lequel le rapport entre silicium et aluminium dans ledit aluminosilicate est compris dans la gamme allant de 0,9 pour 1 à 1,15 pour 1.

15. Utilisation d'aluminosilicate de métal alcalin du type zéolite P ayant un rapport silicium sur aluminium ne dépassant pas 1,33 en tant qu'un matériau porteur pour incorporer du parfum dans une composition détergente et réduire la pression de vapeur du parfum, la quantité de parfum sur ledit aluminosilicate ne dépassant pas 8 % de la masse de celui-ci.

## Revendications (Revendications pour l'(les) Etat(s) contractant(s) suivant(s): ES)

1. Composition détergente sous forme de particules contenant :
de 2 à 60 % en masse d'un ingrédient détergent actif;
de 5 à 60 % en masse d'un aluminosilicate de métal alcalin du type zéolite P ayant un rapport silicium sur aluminium qui n'est pas supérieur à 1,33, et
de 0,1 à 3 % en masse de parfum qui est au moins partiellement absorbé sur ledit aluminosilicate, de telle sorte qu'il y ait une concentration de parfum plus importante sur ledit aluminosilicate que dans le reste de la composition, la
masse de parfum n'étant pas supérieure à 8 % en masse dudit aluminosilicate.

2. Composition détergente sous forme de particules selon la revendication 1, dans lequel la quantité de parfum va de 0,5 à 8 % en masse dudit aluminosilicate.

3. Composition détergente sous forme de particules selon la revendication 1, dans lequel la quantité de parfum va de 1 à 8 % en masse dudit aluminosilicate.

4. Composition sous forme de particules selon la revendication 1, dans lequel la quantité de parfum va de 0,5 à 6 % en masse dudit aluminosilicate.

5. Composition sous forme de particules selon l'une des revendications 1 à 4, dans laquelle le rapport Si sur Al dans ledit aluminosilicate est compris dans la gamme allant de 0,9 pour 1 à 1,15 pour 1.

6. Composition sous forme de particules selon l'une des revendications précédentes, dans laquelle le parfum comprend des matériaux de parfumerie ayant une masse moléculaire supérieure à 150 mais ne dépassant pas 300.

7. Composition selon l'une des revendications précédentes contenant de 10 à 40 % en masse dudit aluminosilicate.

8. Procédé de fabrication d'une composition détergente sous forme de particules comprenant de 2 à 60 % en masse d'ingrédient détergent actif, de 5 à 60 % en masse d'aluminosilicate de métal alcalin du type zéolite P ayant un rapport silicium sur aluminium qui n'est pas supérieur à 1,33, et de 0,1 à 3 % en masse de parfum, ledit procédé comprenant les étapes consistant à :
(i) absorber le parfum sur un aluminosilicate de métal alcalin du type zéolite P ayant un rapport silicium/aluminium qui n'est pas supérieur à 1,33 ;
(ii) ensuite, mélanger ledit aluminosilicate et le parfum absorbé dessus avec les autres ingrédients de la composition comprenant le matériau détergent actif, la quantité dudit parfum étant comprise entre 0,2 et 8 % en masse de l'aluminosilicate dudit type dans la composition.

9. Procédé selon la revendication 8, dans lequel la quantité de parfum va de 0,5 à 8 % en masse dudit aluminosilicate.

10. Procédé selon la revendication 8, dans lequel la quantité de parfum va de 1 à 8 % en masse dudit aluminosilicate.

11. Procédé selon la revendication 8, dans lequel la quantité de parfum va de 0,5 à 6 % en masse dudit aluminosilicate.

12. Procédé selon l'une des revendications 8 à 11 dans lequel le parfum comprend un matériau de parfumerie ayant une masse moléculaire supérieure à 150 mais ne dépassant pas 300.

13. Procédé selon l'une des revendications 8 à 12, dans lequel la quantité dudit aluminosilicate va de 10 à 40 % en masse de la composition.

14. Procédé selon l'une des revendications 8 à 13, dans lequel le rapport entre silicium et aluminium dans ledit aluminosilicate est compris dans la gamme allant de 0,9 pour 1 à 1,15 pour 1.

15. Utilisation d'aluminosilicate de métal alcalin du type zéolite P ayant un rapport silicium sur aluminium ne dépassant pas 1,33 en tant qu'un matériau porteur pour incorporer du parfum dans une composition détergente et réduire la pression de vapeur du parfum, la quantité de parfum sur ledit aluminosilicate ne dépassant pas 8 % de la masse de celui-ci.
